# EUROPEAN PATENT SPECIFICATION

(11) **EP 1 855 544 B1**
(45) Date of publication and mention of the grant of the patent: **19.01.2011**
(21) Application number: 06716667.8
(22) Date of filing: 23.02.2006
(51) Int. Cl.: A23L 1/0524

(54) **GELATION OF ANIONIC POLYSACCARIDES USING PROTEIN HYDROLYSATES**
GELIERUNG ANIONISCHER POLYSACCHARIDE UNTER VERWENDUNG VON PROTEINHYDROLYSATEN
GELATION DE POLYSACCHARIDES ANIONIQUES AU MOYEN D'HYDROLYSATS DE PROTEINES

(30) Priority: 24.02.2005 EP 05075452
(43) Date of publication of application: 21.11.2007
(73) Proprietor: Unilever NV, 3013 AL Rotterdam (NL); Unilever PLC, London EC4Y 0DY (GB)
(72) Inventor: VAN DE VELDE, Freddie, NL-6715 HJ Ede (NL)
(74) Representative: Swinkels, Bart Willem
(86) International application number: PCT/NL2006/050034
(87) International publication number: WO 2006/091101

(56) References cited:
- TOSA T ET AL: "IMMOBILIZATION OF ENZYMES AND MICROBIAL CELLS USING CARRAGEENAN AS MATRIX" BIOTECHNOLOGY AND BIOENGINEERING, INTERSCIENCE PUBLISHERS, LONDON, GB, vol. 21, 1979, pages 1697-1709, XP009001718 ISSN: 0006-3592
- TAGUCHI T ET AL: "Swelling behavior of hyaluronic acid and type II collagen hydrogels prepared by using conventional crosslinking and subsequent additional polymer interactions" JOURNAL OF BIOMATERIALS SCIENCE POLYMER EDITION, vol. 13, no. 1, 2002, pages 43-52, XP009074898 ISSN: 0920-5063

## Description

### FIELD OF THE INVENTION

The present invention relates to the field of gelling and/or viscosifying hydrocolloid compositions. More in particular, it relates to the use of peptide materials as cross-linking agent for gelling and/or viscosifying aqueous anionic polysaccharide compositions. Accordingly, hydrocolloid compositions comprising anionic polysaccharides as well as an amount of peptide material are provided by the present invention, as well as gelled and/or viscosified aqueous systems, including e.g. foodstuffs, comprising said hydrocolloid compositions.

### BACKGROUND OF THE INVENTION

For applications of hydrocolloids, such as proteins and polysaccharides, in the pharmaceutical, cosmetic and food industries, the interactions between the hydrocolloid molecules are of major relevance. Many hydrocolloids are able to viscosify aqueous systems and/or to form hydrogels. Hydrogels are water-containing networks of hydrocolloids showing solid-like behaviour with characteristic strength, dependent on their concentration, and hardness and brittleness dependent on the structure of the hydrocolloid(s) present.

In its most general definition a gel is described as a mixture of at least two components one of which is a liquid, present in a substantial amount. Moreover, gels show solid or solid-like behaviour. In order to display this solid or solid-like behaviour, the minor component has to form a space-filling three-dimensional network. Because of the coexistence of this network structure within a liquid phase, a gel behaves like an elastic solid at low stress values, but above a finite yield stress, they turn into a viscous liquid.

Hydrogels are typically prepared by dispersing and hydrating hydrocolloids, optionally heating and cooling. Subsequently the hydrocolloids form a three-dimensional polymer network with chemical or physical cross-links between various polymeric chains, resulting in a macroscopic hydrogel. Such cross-links between polymer chains can be any chemical or physical interaction, including covalent bonding, ionic bonding, hydrogen bonding, hydrophobic associations, dipole-dipole interactions and van der Waals interactions. Therefore, gels can be divided into two groups depending on the types of interactions that hold the network structure together.

Chemical gels are formed through covalent cross-linking of dilute solutions of polymers or inorganic oxides, and because of the covalent nature of the network, their formation is thermally irreversible. An example of covalent cross-linking of polycarboxylic acids can be found in FR 2 752 843. In said document, a cross-linking agent comprising at least two amine groups, e.g. polylysine or polyornithine is used. The process comprises combining the polycarboxylic acids, an activating agent and the cross-linking agent under such conditions that covalent bonds between the polycarboxylic acids and the cross-linking agent are formed. The cross-linked co-polymers thus formed are non-soluble in aqueous systems and are obtained as a precipitate. Formation of a (chemical) gel using these cross-linked co-polymers is not disclosed in FR 2 752 843.

In physical gels, the subunits constituting the network structure are held together by noncovalent interactions. Many gels formed by polymers, proteins, surfactants, small organic molecules, and even mineral clays, belong to this class.

In general formation of physical gels based on anionic polysaccharides relies on cationic cross-linking agents to crosslink the chains having anionic functional groups, such as -SO₃H and -COOH, into a network.

The most commonly used cross-linking agents include mono- or multivalent metal ions. For most types of anionic polysaccharides divalent cations are more effective in promoting gelation, whereas other types are more effectively gelled using monovalent metal cations. Trivalent metal cations are often found to cause precipitation. Both gel strength and melting temperature increase with increasing metal cation concentration.

The use of organic polymer compounds having one or more cationic residues for cross-linking anionic polysaccharides has been described. In US 4,996,150 biocatalyst systems encapsulated in beads comprised of anionic polysaccharide and cationic polymer are described. The cationic polymer can be any polyalkane amine and/or polyalkane imine.

US 4,138,292 describes immobilizing enzymes and microorganisms in a gel matrix obtained by contacting an aqueous solution of a sulphated polysaccharide with ammonium ions, a metal ion, a water-soluble amine or a water-miscible organic solvent. According to this document representative examples of such polysaccharides include carrageenan, furcellaran and cellulose sulphate. Suitable organic amines for gelling or solidifying the polysaccharide include alkylenediamines of 1-20 carbon atoms, hydroxamates, hydrazides alkyl-esters or amides of basic amino acids, peptides and polyalkylene imines. Suitable examples of peptides that can be applied according to US 4,138,292 comprise lysyl-lysine, histidyl-lysine, histidyl-arginine, histidyl-arginyl-lysine and polylysine

Tosa et al. ("Immobilization of enzymes and microbial cells using carrageenan as matrix", Biotechnology and bioengineering, 21 (10), p.1697-1709, Oct 1979) describe the results of a study in which immobilization of enzymes and microbial cells in κ-carrageenan was investigated. Gel formation was induced by contacting the κ-carrageenan with metal ions, amines, amino acid derivatives and water-miscible solvents. The authors report that the following amino acids and amino acid derivatives were capable of inducing gelations: agmatine, histamine, L-ornithine, L-lysine, δ-hydroy-L-lysine, L-lysine hydroxamate, 1-histidine hydroxamate, DL-histidine hydrazide, L-tryptophan hydroxamate, S-2-aminoethyl-L-cysteine.

Taguche et al. ("Swelling behavior of hyaluronic acid and type II collagen hydrogels prepared by using conventional crosslinking and subsequent additional polymer interactions", Journal of biomaterials science. Polymer edition, 13 (1), p.43-52, Jan 2002) describe a study in which polymer-polymer interactions were introduced in covalently crosslinked composite matrices consisting of an anionic polysaccharide, hyaluronic acid (HyA) and a cationic polypeptide, type II collagen. The matrices were covalently crosslinked using a water soluble carbodiimide.

The specific properties of a hydrogel, i.e. with regard to viscosity, elasticity, hardness, appearance, etc., is affected by various factors including the choice of polysaccharide, the choice of cross-linking agent, pH, solids contents, etc. Also the need to control the gelation rate is well-recognised affecting e.g. mechanical strength and the pore size of the gel obtained. For many applications such as in biomedical, pharmaceutical, food and cosmetic formulations, the necessary/preferred rate of gelation falls within relatively narrow parameters. Factors that have been reported to control gelation rate include for example, the solubility of the cation containing compounds; cation concentration; mixture/ratio of cation containing compounds; polymer concentration; gelation temperature; application of shear, etc.

### SUMMARY OF THE INVENTION

The present inventors have as a result of extensive research and experimentation, found that aqueous compositions comprising anionic polysaccharides can suitably be gelled and/or thickened using 'cross-linking' peptides. More particularly, hydrocolloid compositions comprising an anionic polysaccharide and peptides are provided, wherein said peptides are typically obtained by hydrolysing protein compositions and optionally subsequently isolating or purifying a cationic fraction therefrom. Furthermore, the use of the present hydrocolloid systems as a thickening and/or gelling agent is disclosed.

Compositions comprising polysaccharides and proteins for microencapsulation of particulate materials by complex coacervation are well known in the art of colloid chemistry. In general coacervation refers to the phenomenon of phase separation of colloid dispersions into two liquid phases, i.e. a phase poor in polymers (colloids) and rich in solvent and a phase comprising coacervate droplets concentrated in polymers. Coacervation of colloids can be brought about in a number of ways, including changing the temperature or the pH or addition of a second polymeric compound. Typically the tendency for polymeric complex formation and phase separation to occur, increases with charge density of the polymers and polymer molecular weight (MW). An increase in polymer MW reduces the charge density required for coacervation.

The preparation of drug delivery microcapsules by complex formation of poly(L-lysine) and alginate or carrageenan has been described by Thu et al ("Alginate polycation

microcapsules 1. Interaction between alginate and polycation", Biomaterials, 17 (1996), 1031-1040) and Girod et al ("Polyelectrolyte complex formation between iota-carrageenan and poly(L-lysine) in dilute aqueous solutions: a spectroscopic and conformational study", Carbohydrate Polymers 55 (2004) 37-45) respectively. In their work Girod et al studied the interaction between poly-L-lysine (PLL) and iota-carrageenan in the concentrations domain where the complexes formed are soluble. Studies on phase-separated systems comprising PLL and iota-carrageenan, especially regarding the effect of parameters such as pH, ionic strength, charge densities and molecular weight on the extent of the phase separation, had been described before.

Complex formation of casein hydrolysates with sugar beet pectin and/or low-saccharification starch has been described by Tokaev et al ("Study of complex formation by food proteins by gel filtration and ultracentrifugation", FSTA database 89-1-10-a0021; IFIS (1987)). More particularly Tokaev et al. have studied the formation of these soluble complexes using using gel filtration, as well as the stability of emulsions comprising them by sedimentation analyses. Since, the complexes formed, can be separated by gel filtration, inevitably, they exist as discrete elements (or coacervates) within the aqueous system. According to Tokaev et al. the casein hydrolysate contained 20-25 kDa peptide fragments.

The systems described by Girod et al. and Tokaev et al. cannot form gels. Thickening and/or gelling of the present hydrocolloid compositions in aqueous systems do not involve macroscopic phase separation.

### DETAILED DESCRIPTION OF THE INVENTION

Thus, the present invention in a first aspect relates to gelling and/or thickening hydrocolloid compositions comprising, based on the dry weight of said compositions, 1-95 wt% of anionic polysaccharides and 1-95 wt% of cross-linking peptides having a molecular mass within the range of 0.3-12 kDa, wherein said peptides comprise a total amount of lysine, histidine and arginine of between 2.5-40 mol%, based on the total amount of amino acid residues comprised in the peptides.

The terms 'thickened' and 'thickening' as used herein refer to the increased viscosity of an aqueous system or composition and to increasing the viscosity of (or viscosifying) aqueous compositions, respectively. Hence these terms are used herein interchangeably with the terms 'viscosified' and 'viscosifying' respectively.

The term 'anionic polysaccharide', as used herein refers to any type of polysaccharide comprising functional groups that can carry one or more negative charges in aqueous systems, e.g. carboxyl groups, phosphate groups and/or sulphate groups. The anionic polysaccharides can be derived from any source and/or may be obtained by modification, e.g. derivatization, of a polysaccharide. Preferably the anionic polysaccharides according to the present invention is selected from the group comprising carrageenans, alginates, agar, pectins, modified pectins, gellan gum, xanthan gum, furcellaran, cellulose derivatives, particularly carboxymethyl cellulose (CMC) and cellulose sulphate, dextran sulphate, modified starches, exopolysaccharides and mixtures thereof.

According to another embodiment the present composition comprises anionic polysaccharides containing on average not more than 1.5 of the aforementioned (anionic) functional groups per monosaccharide, more preferably not more than 1.0.

The present inventor has found that any anionic polysaccharide that can be used to viscosify and/or gellate aqueous systems in the presence of metal cations, can suitably do so as well in the presence of cross-linking peptides according to the present invention. Therefore, according to an even more preferred embodiment the present gelling and/or thickening hydrocolloid composition comprises anionic polysaccharide is selected from carrageenans, CMC, pectins, xanthan gum, gellan gum, agar agar and mixtures thereof, still more preferably from carrageenans, CMC, xanthan gum, gellan gum, agar agar and mixtures thereof.

According to a most preferred embodiment a gelling hydrocolloid composition is provided wherein the anionic polysaccharide is selected from κ-carrageenan, τ-carrageenan, τ/κ-hybrid carrageenan, gellan gum, and mixtures thereof.

It is generally known that systems composed of more than one polysaccharide exhibit a unique complexity of non-additive properties that result in thickening efficiency, or hybrid rheologies and textures with sensory and processing advantages. Hence, the hydrocolloid composition may suitably comprise mixtures of the aforementioned anionic polysaccharides and non-ionic or cationic polysaccharides.

The hydrocolloid composition provided by the present invention comprises at least 1 wt% of anionic polysaccharides, based on the dry weight of said composition, even more preferably at least 5 wt%, still more preferably at least 10 wt% and most preferably at least 25 wt%. Preferably the amount of the anionic polysaccharides does not exceed, based on the dry weight of the composition, 95 %, even more preferably it does not exceed 90 wt%, most preferably it does not exceed 75 wt%.

As mentioned herein before, the present hydrocolloid composition comprises 1-95 wt% of cross-linking peptides having a molecular mass within the range of 0.3-12 kDa, said peptides comprising a total amount of lysine, histidine and arginine of between 2.5-40 mol%. According to a preferred embodiment said peptides are obtained by hydrolyzing a protein composition, optionally followed by isolating therefrom a cationic fraction. Protein compositions originating from any source may suitably be used according to the present invention.

Hence, according to a particularly preferred embodiment, the present cross-linking peptides are comprised in a hydrolyzed protein composition or in a cationic fraction isolated therefrom, wherein said protein composition comprises one or more proteins selected from cereal proteins, egg white proteins, milk proteins, including caseines and, whey proteins), soy proteins, pea proteins, potato proteins, corn proteins, gelatines and mixtures thereof.

The aforementioned proteins may be hydrolyzed in any way known to the skilled professional, which usually includes enzymatic treatment, e.g. with trypsin or pepsin, or chemical hydrolysation, e.g. using hydrochloric acid, followed by neutralization with hydroxide. According to the present invention, it is particularly preferred to enzymatically hydrolyze the protein composition, most preferably using pepsin. This enzyme is known to generate suitable cationic fragments from proteins such as caseines and lactoferrine.

According to the present invention, it is essential that the protein is not hydrolyzed completely, i.e. into free amino acids. As mentioned herein before the peptides have a molecular mass within the range of 0.3-12 kDa, in an even more preferred embodiment said molecular mass is within the range of 0.5-10 kDa. In a most preferred embodiment said molecular mass is within the range of 0.5-8.0 kDa.

Unlike any of the prior art disclosures relating to the use of synthetic peptides for gelling anionic polysaccharides, the present cross-linking peptides are not solely comprised of amino acid residues having a cationic side chain. More specifically, the present cross-linking peptides comprise a total amount of lysine, histidine and arginine of 2.5-40 mol%, based on the total amount of amino acid residues comprised in said peptides, preferably said amount ranges from 2.5-25 mol%, more preferably from 5-25 mol%, most preferably from 10-25 mol%.

Since the present cross-linking peptides are typically obtained by hydrolyzing a protein composition, said cross-linking peptides typically do not comprise any amino acid residues other than those that can be coded for by nucleic acid sequences in nature, i.e. other than those selected from Ala, Arg, Asn, Asp, Cys, Glu, Gln, Gly, His, Ile, Leu, Lys, Met, Phe, Pro, Ser, Thr, Trp, Tyr and Val. Thus, according to a particularly preferred embodiment the present cross-linking peptides comprise at least 90 mol%, more preferably at least 95 mol%, still more preferably at least 98 mol%, most preferably at least 99 mol% of amino acid residues that can be coded for by one or more nucleic acid sequences.

The hydrocolloid composition may comprise other proteinaceous materials in addition to the 1-95 wt% of cross linking peptides defined herein before. The term 'proteinaceous material' as used herein, refers to any type of material comprising proteins, peptides and/or free amino acids.

Typically, instead of applying a protein hydrolysate comprising the present peptides as such, a cationic fraction of a protein hydrolysate may be used according to the present invention. Said fraction may be purified or isolated from the hydrolysate using any conventional method known to the skilled professional. The term 'cationic fraction' as used herein, refers to a fraction characterized by a relatively high content, in comparison to the starting material, of amino acid residues having cationic side chains, i.e. histidine, arginine and lysine. Typically, the present cationic fractions of protein hydrolysates comprise a total amount of lysine, histidine and arginine of between 10-25 mol%. Suitable methods of purifying or isolating a cationic fraction include preparative ion-exchange chromatography, electro membrane filtration, electrodialysis.

The hydrocolloid composition provided by the present invention comprises, based on the dry weight of said composition, at least 1.0 wt%, preferably at least 5.0 wt% of the cross-linking peptides as defined herein before, even more preferably at least 10 wt%, most preferably at least 25 wt%. Preferably the amount of said cross-linking peptides does not exceed 95 wt%, based on the dry weight of the composition, even more preferably it does not exceed 90 wt%, most preferably it does not exceed 75 wt%.

According to the present invention the anionic polysaccharides and cross-linking peptides are applied in a weight ratio ranging from 1.0:15.0 to 15.0:1.0, more preferably from 1.0:10.0 to 10.0:1.0, even more preferably from 1.0:5.0 to 5.0:1.0.

Another preferred embodiment of the present invention relates to a hydrocolloid composition as defined herein before wherein the weight ratio of said anionic polysaccharides and said peptides is chosen such that the composition comprises lysine, histidine and arginine in an amount of 0.01-75 mmol per gram of anionic polysaccharides, more preferably in an amount of 0.1-25 mmol per gram of anionic polysaccharides, most preferably 1-20 mmol per gram of said polysaccharides.

According to a particularly preferred embodiment the present hydrocolloid compositions do not comprise any coupling agents, such as those traditionally used in the synthesis of peptides. More particularly, the hydrocolloid compositions of the invention do not comprise any carbodiimides, quinoline derivatives and/or mixed anhydrides, such as chloroformates.

Another aspect of the present invention relates to a process of preparing a hydrocolloid composition, which process comprises hydrolyzing a protein composition to such a degree that peptides having an average molecular mass within the range of 0.3-12 kDa are obtained, typically to a degree of hydrolysis (DH) of between 5-40 %, and combining said hydrolyzed protein composition with anionic polysaccharides in a ratio ranging from 1.0:15.0 to 15.0:1.0. The term "degree of hydrolysis" (DH) refers to the percentage of peptide bonds cleaved. The method utilized to determine DH is the TNBS colorimetry method described in Adler-Nissen, J., "Determination of the Degree of Hydrolysis of Food Protein Hydrolysates by Trinitrobenzenesulfonic Acid", J. Agric. Food Chem., Vol. 27, No. 6, 1979, pg. 1256-62. The further a protein is hydrolyzed (higher DH), the lower the average molecular weight; the peptide profile changes accordingly. Preferably the degree of hydrolysis is between 7-30 %. As mentioned herein before, it is preferred that said protein composition is hydrolyzed enzymatically, most preferably using pepsin. The conditions and incubation time necessary for achieving the desired degree of hydrolysis can be easily determined by the skilled person.

According to a preferred embodiment said process further comprises isolating a cationic fraction from the hydrolyzed protein and combining said fraction with the present anionic polysaccharides.

Still another aspect of the present invention relates to a hydrocolloid composition obtainable by the process described here above.

Since, according to the present invention, the anionic polysaccharide polymers are cross-linked by peptides, the present hydrocolloid composition is characterised by the fact that it can be applied using low concentrations of salts. Hence according to a preferred embodiment the composition comprises not more than 10 mmol per g of anionic polysaccharides of metal cations selected from Na⁺, K⁺ and Ca²⁺, more preferably not more than 6 mmol per g of anionic polysaccharides.

According to the present invention gelled or thickened aqueous compositions are provided comprising the present hydrocolloid composition. More particularly, one aspect of the invention relates to gelled or thickened aqueous compositions comprising, based on the total weight of the composition, 0.01-10 wt% of anionic polysaccharides and 0.01-40 wt% of cross-linking peptides having a molecular mass within the range of 0.3-12 kDa, wherein said peptides comprise a total amount of lysine, histidine and arginine of between 2.5-40 mol%, based on the total amount of amino acid residues comprised in said peptides.

According to a preferred embodiment, the present gelled or thickened aqueous systems are prepared by combining the hydrocolloid composition with said aqueous system. Preferably the pH of the aqueous system ranges from 3.0-8.5, more preferably from 4.0-8.0, most preferably 4.0-7.0. The aqueous system may be heated and/or shear forces may be applied to it when adding the hydrocolloid composition in order to improve the hydration of the anionic polysaccharides. If the aqueous dispersion of the hydrocolloid composition is allowed to cool and/or if no more shear force is applied the aqueous system will start to viscosify or solidify.

The present hydrocolloid compositions can suitably be used for incorporation into various types of foodstuffs for providing texture, structure, viscosity, elasticity and/or stability. Therefore another aspect of the present invention relates to a foodstuff comprising, based on the total weight of the foodstuff, 0.01-95 wt%, of a gelled or thickened aqueous hydrocolloid composition as defined herein before. Typically the foodstuff is a water continuous foodstuff. Suitable examples include desserts, dressings, ice cream, and beverages.

Since, according to the present invention, gelled or thickened compositions are suitably prepared without the addition of metal cations, said hydrogels may advantageously be applied in low-salt foodstuffs, i.e. foodstuffs comprising little or no NaCl, such as those typically applied in diets used for persons suffering from heart failure, impaired liver function, high blood pressure, acute and chronic kidney disease, etc. Thus, a particularly preferred embodiment of the invention relates to foodstuffs comprising the present aqueous hydrocolloid composition, said foodstuff comprising not more than 30 mM, preferably not more than15 mM of metal cations selected from Na⁺, K⁺ and Ca²⁺.

The present hydrocolloid compositions may suitably be used for immobilizing enzymes and/or microorganisms that are used to catalyze various conversions, by entrapping them within a gel matrix. Alternatively the present hydrocolloid composition may be used to prepare microcapsules comprising therapeutic and/or diagnostic agents, e.g. for controlled drug release. Hence, another aspect of the present invention relates to an encapsulate comprising an active ingredient contained in a matrix comprising 0.5-95 wt% of anionic polysaccharides and 0.5-95 wt% of peptides having a molecular mass within the range of 0.3-12 kDa, wherein said peptides comprise a total amount of lysine, histidine and arginine of between 2.5-40 mol%, based on the total amount of amino acid residues comprised in said peptides. Active ingredients that can advantageously be encapsulated in a matrix according to the present invention include medicaments, vaccines, enzymes, hormones, oligonucleotides, vitamins, etc.

In still an alternative embodiment the present gelled or thickened aqueous hydrocolloid composition can be used for preparing a cosmetic composition or a therapeutic composition for topical application to the skin. Hence, still another aspect of the present invention relates to a aqueous hydrocolloid compositions as defined herein before comprising a therapeutic or cosmetic agent.

Another aspect of the present invention relates to the use of peptides having a molecular mass within the range of 0.3-12 kDa and comprising a total amount of lysine, histidine and arginine of between 2.5-40 mol%, based on the total amount of amino acid residues comprised in the peptides, as defined herein before, for improving the gelling and/or viscosifying characteristics of anionic polysaccharides.

The invention is further illustrated in the following examples which are not intended to be in any way limiting to the scope of the invention as claimed.

### EXAMPLES

### Example 1

A commercially available protein hydrolysate, Hyfoama 66 (ex Quest B.V. Naarden, the Netherlands), was added to aqueous solutions of τ-carrageenan (0.5 % w/v) at different pH values (pH 5.8, pH 7.0 and pH 8.0) in an amount of 25 mg/ml. The characteristics (i.e. molecular weight distribution of the fragments and the amount (mol%) of histidine, arginine and lysine comprised in the hydrolysate) of the Hyfoama 66 were determined in a further experiment. These results are presented in example 2. The Hyfoama 66 was dissolved in the carrageenan solutions by heating to 60 °C. The gel formation in Eppendorf (2 ml) tubes at room temperature was checked visually.

It was observed that τ-carrageenan forms gels at Hyfoama 66 concentrations of 25 mg/ml at pH 5.8 and pH 7.0. and pH 8.0.

### Example 2

Cationic fractions were prepared from 4 protein hydrolysates, Hyfoama 66, sodium-caseinate, lactoferrin and egg white protein (EWP). The effect of the peptide fractions on carrageenan gelation was tested. Furthermore the cationic peptide fractions were analysed on amino acid composition and on molecular weight distribution by GPC.

10 grams of sodium-caseinate and EWP and lactoferrin were hydrolysed with pepsin (5% protein, pH 3) by incubating for 4 hours at 37°C. Pepsin was inactivated by adjusting the pH to 7.0. Dialysis (MWCO 100) of the hydrolysates was carried out before chromatographic isolation of the cationic fraction and after the isolation of this fraction. Dialysis was continued until the conductivity [µS/cm] reached a stable value. The desalted hydrolysates and cationic fractions thereof were frozen and lyophilized. The three desalted newly prepared hydrolysates and Hyfoama 66 were fractionated. Desalted hydrolysates were dissolved in 50 mM Na-Acetate buffer pH 4.5. Non-soluble material was removed by centrifugation and filtration (0.45 µm) and the filtrate was applied onto a column packed with HiTrap SP (Amersham) by the use of the chromatographic system Åkta (Amersham). After washing of the column with the same buffer, the cationic fraction was eluted with 50 mM Na-Acetate buffer pH 4.5 containing 1M NaCl.

The molecular weight distribution was determined by the use of size exclusion chromatography (NIZO food research-method: Visser et al in Journal of Chromatography 599 (1992) 205-209). With the use of marker proteins with known molecular masses the elution times corresponding with protein-fragments having molecular masses of 5 and 10 kDa were determined. These elution times were used to divide the fragments into three classes, > 10 kDa, between 10 and 5 kDa and <5 kDa. Table 1 shows the distribution in these three classes of the different hydrolysates and cationic fractions thereof. The cationic fractions obtained from the hydrolysates were all enriched in fragments having a molecular mass > 5 kDa.

**Table 1: Molecular mass distribution of hydrolysates and cationic fractions thereof.**

| **Sample** | **Area (% of total area)** | | |
|---|---|---|---|
| | **Area> 10 kDa** | **10 kDa>area>5 kDa** | **Area < 5 kDa** |
| **Sodium-caseinate hydrolysates** | 26 | 18 | 56 |
| **Lactoferrin hydrolysates** | 33 | 18 | 49 |
| **EWP hydrolysates** | 48 | 13 | 39 |
| **Hyfoama 66** | 39 | 21 | 40 |
| **Sodium-caseinate cationic fraction** | 18 | 38 | 44 |
| **Lactoferrin cationic fraction** | 39 | 28 | 33 |
| **EWP cationic fraction** | 53 | 18 | 28 |
| **Hyfoama 66 cationic fraction** | 57 | 22 | 22 |

Determination of the amino acid composition in hydrolysates and cationic fraction thereof was carried out by Ansynth service B.V. according to the acid hydrolysis method. The percentage cationic amino acids (Lys, His and Arg) in the hydrolysates and the cationic fractions thereof are shown in Table 2.

**Table 2: Mol-percentage cationic amino acids in the different protein sources and in the cationic fractions thereof.**

| | **mol% Lys-His-Arg/total** | |
|---|---|---|
| **Sample** | **Protein** | **cationic fraction** |
| **Hyfoama** | 5.3 | 10.4 |
| **NaCas** | 11.7 | 18.9 |
| **Kip-eiwit** | 12.3 | 17.1 |
| **Lactoferrine** | 15.9 | 18.8 |

The effect of the prepared cationic fractions on the gelation behaviour of anionic polysaccharides was evaluated with kappa and iota carrageenan. The gel formation in Eppendorf (2 ml) tubes at room temperature was checked visually, by decanting. In Table 3 the results of the evaluation of the effect of cationic peptides on the gelation of carrageenans are given. With the concentration range applied in all cases gelation could be observed as a result of the addition of the cationic fractions. Depending on the concentration cationic fraction difference in gel characteristics were found. Differences in appearance were found. The cationic fractions derived from EWP and lactoferrin caused the formation of a turbid gel, whereas cationic fractions of Hyfoama 66 and sodium-caseinate resulted in transparent gels.

**Table 3: Effect of different cationic peptide fractions on the gelation behaviour of kappa carrageen and iota carrageen (0.5% (w/v)).**

| **Cationic fraction** | **Concentration cationic fraction [mg]/[mL] kappa carrageenan 0.5%** | | |
|---|---|---|---|
| | 10 | 25 | Remarks |
| **Hyfoama** | + | +/- | Transparent |
| **Na-Cas** | + | - | Transparent |
| **EWP** | + | ++ | Turbid |
| **Lactoferrin** | ++ | ++ | Turbid |
| | 10 | 25 | Remarks |
| **Hyfoama** | + | + | Transparent |
| **Na-Cas** | + | + | Transparent |
| **EWP** | + | + | Turbid |
| **Lactoferrin** | + | + | Turbid |

| | | | |
|---|---|---|---|
| Score: - no gelation +/- weak gelation/precipitation + weak gel ++ strong gel | | | |

### Example 3

A cationic fraction of lactoferrin was applied to evaluate the effect on other anionic polysaccharides. A series of five anionic polysaccharide preparations were evaluated. The gel formation in Eppendorf (2 ml) tubes at room temperature was checked visually. The results are shown in Table 4. It was observed that gelation of τ/κ-hybrid carrageenan, xanthan and gellan gum could be induced by the addition of the cationic peptide fraction of the lactoferrin hydro lysates.

**Table 4: Effect of lactoferrin cationic peptide fraction on the gelation behaviour of different anionic polysaccharides (0.5% (w/v)).**

| **Polysaccharide** | **10mg/mL** | **50mg/mL** | **Remarks** |
|---|---|---|---|
| **1. τ/κ-hybrid carrageenan** | V | + | Turbid weak gel |
| **2. Xanthan** | V | +V | Increase in viscosity |
| **3. LM Pectin** | - | V | |
| **4. Gellan gum** | **++** | **++** | Transparent gel |
| **5. Agar-Agar** | Gel particles | Gel particles | |

| | | | |
|---|---|---|---|
| - no gelation +/- weak gelation/precipitation + weak gel ++ strong gel V Increase in viscosity | | | |

### Example 4

Intact proteins and synthetically prepared poly-L-lysine (Sigma-Aldrich, Zwijndrecht, the Netherlands) were applied to evaluate their effect on κ-, τ- and λ-carrageenan (0.5% (w/w). The behaviour of these mixtures was inspected visually in Eppendorf (1.5 ml) tubes at room temperature. The results are shown in Table S.Complex formation was observed in al cases. Complex formation upon mixing of the anionic carrageenan and the cationic peptide or poly-L-lysine resulted in the formation of a cloudy, whitish or reddish in the case of cytochrome c, material in the solution.

**Table 5: Effect of peptides and poly-L-lysine on the behaviour of carrageenans (0.5% (w/v)).**

| Protein | Carrageenan | | |
|---|---|---|---|
| | κ | τ | λ |
| cytochrome c | Complex formation | Complex formation | Complex formation |
| lysozyme | Complex formation | Complex formation | Complex formation |
| poly-L-lysine 13 kDa | Complex formation | Complex formation | Complex formation |
| poly-L-lysine 405 kDa | Complex formation | Complex formation | Complex formation |

## Claims

1. Hydrocolloid composition comprising, based on the dry weight of said composition, 1-95 wt% of anionic polysaccharides and 1-95 wt% of peptides having a molecular mass within the range of 0.3-12 kDa, wherein said peptides comprise a total amount of lysine, histidine and arginine of between 2.5-40 mol%, based on the total amount of amino acid residues comprised in the peptides.

2. Hydrocolloid composition according to claim 1, wherein the anionic polysaccharide is selected from carrageenans, alginates, agar, pectins, modified pectins, gellan gum, xanthan gum, furcellaran, cellulose derivatives, particularly carboxymethyl cellulose (CMC) and cellulose sulphate, dextran sulphate, modified starches, exopolysaccharides, and mixtures thereof;

3. Hydrocolloid composition according to claim 1 or 2, wherein the weight ratio of said anionic polysaccharides and said peptides ranges from 1:15 to 15:1.

4. Hydrocolloid composition according to any one of the preceding claims wherein the weight ratio of said anionic polysaccharides and said peptides is chosen such that the composition comprises lysine, histidine and arginine in an amount of 0.01 - 75 mmol per gram of anionic polysaccharides.

5. Hydrocolloid composition according to any one of the preceding claims comprising not more than 10 mmol per g of anionic polysaccharides of metal cations selected from Na⁺, K⁺ and Ca²⁺.

6. Hydrocolloid composition according to any one of the preceding claims, wherein the peptides have a molecular mass within the range of 0.5- 8.0 kDa.

7. Hydrocolloid composition according to any one of the preceding claims wherein the peptides comprise a total amount of lysine, histidine and arginine of between 5-25 mol%, based on the total amount of amino acid residues comprised in said peptides.

8. Hydrocolloid composition according to any of the preceding claims, wherein the peptide is comprised in a hydrolyzed protein composition.

9. Hydrocolloid composition according to claim 8, wherein the protein composition comprises one or more proteins selected from cereal proteins, egg white proteins, milk proteins, soy proteins, pea proteins, potato proteins, corn proteins, gelatines and mixtures thereof.

10. Hydrocolloid composition according to any of the preceding claims wherein the anionic polysaccharide is selected from κ-carrageenan, τ-carrageenan, τ/κ-hybrid carrageenan, gellan gum, and mixtures thereof.

11. Gelled or thickened aqueous composition comprising, based on the total weight of the composition, 0.01-10 wt% of anionic polysaccharides and 0.01-40 wt% of peptides having a molecular mass within the range of 0.3-12 kDa, wherein said peptides comprise a total amount of lysine, histidine and arginine of between 2.5-40 mol%, based on the total amount of amino acid residues comprised in the peptides and wherein the weight ratio of anionic polysaccharides and the peptides ranges from 1:15 to 15:1.

12. Foodstuff comprising 0.01-95 wt%, based on the total weight of the foodstuff, of a gelled or thickened aqueous composition as defined in claim 11.

13. Foodstuff according to claims 12, wherein the foodstuff comprises not more than 30 mM of metal cations selected from Na⁺, K⁺ and Ca²⁺.

14. Encapsulate comprising an active ingredient contained in a matrix comprising 0.5-95 wt% of anionic polysaccharides and 0.5-95 wt% of peptides having a molecular mass within the range of 0.3-12 kDa, wherein said peptides comprise a total amount of lysine, histidine and arginine of between 2.5-40 mol%, based on the total amount of amino acid residues comprised in said peptides.

15. Use of peptides having a molecular mass within the range of 0.3-12 kDa and comprising a total amount of lysine, histidine and arginine of between 2.5-40 mol%, based on the total amount of amino acid residues comprised in the peptides, for improving the gelling and/or viscosifying characteristics of anionic polysaccharides.

16. Process of preparing a hydrocolloid composition, which process comprises hydrolyzing a protein composition to such a degree that peptides having an average molecular mass within the range of 0.3-12 kDa are obtained and combining said hydrolyzed protein composition with anionic polysaccharides in a ratio ranging from 1.0:15.0 to 15.0:1.0.

17. Hydrocolloid composition comprising a hydrolyzed protein composition and anionic polysaccharides in a ratio ranging from 1.0: 15.0 to 15.0 : 1.0, said hydrolyzed protein composition having an average molecular mass within the range of 0.3-12 kDa and said hydrocolloid composition being obtainable by the process according to claim 16.

## Patentansprüche

1. Hydrokolloid-Zusammensetzung, welche, basierend auf dem Trockengewicht von der Zusammensetzung, 1 bis 95 Gewichtsprozent von anionischen Polysacchariden und 1 bis 95 Gewichtsprozent von Peptiden, welche eine Molekularmasse innerhalb des Bereiches von 0,3 bis 12 kDa haben, enthält, wobei die Peptide eine Gesamtmenge von Lysin, Histidin und Arginine zwischen 2,5 bis 40 Mol % enthalten, und zwar basierend auf der Gesamtmenge von Aminosäureresten, welche in den Peptiden enthalten sind.

2. Hydrokolloid-Zusammensetzung nach Anspruch 1, bei welcher das anionische Polysaccharid aus Karragenans, Alginat, Agar, Pektin, modifiziertem Pektin, Gelan-Gummi, Xanthan-Gummi, Furkellaran, Zellulose-Derivaten, insbesondere Karboxylmethyl-Zellulose (CMC)und Zellulose-Sulfat, Dextran-Sulfat, modifizierte Stärken, Exopolysacchariden und Mischungen daraus ausgewählt ist.

3. Hydrokolloid-Zusammensetzung nach Anspruch 1 oder 2, bei welcher das Gewichtsverhältnis von den anionischen Polysacchariden und den Peptiden in einem Bereich zwischen 1:15 bis 15:1 ist.

4. Hydrokolloid-Zusammensetzung nach einem der vorhergehenden Ansprüche, bei welchem das Gewichtsverhältnis von den anionischen Polysacchariden und den Peptiden derart gewählt ist, dass die Zusammensetzung Lysin, Histidin und Arginin in einer Menge von 0,01 bis 75 mmol pro Gramm von anionischen Polysacchariden enthält.

5. Hydrokolloid-Zusammensetzung nach einem der vorhergehenden Ansprüche, welche nicht mehr als 10 mmol pro Gramm von anionischen Polysacchariden von Metallkationen enthält, welche aus Na⁺, K⁺ und Ca²⁺ ausgewählt sind.

6. Hydrokolloid-Zusammensetzung nach einem der vorhergehenden Ansprüche, bei welchem die Peptide eine Molekularmasse innerhalb des Bereiches von 0,5 bis 8,0 kDa haben.

7. Hydrokolloid-Zusammensetzung nach einem der vorhergehenden Ansprüche, bei welchem die Peptide eine Gesamtmenge von Lysin, Histidin und Arginine zwischen 5 bis 25 Mol % enthalten, und zwar basierend auf der Gesamtmenge von Aminosäureresten, welche in den Peptiden enthalten sind.

8. Hydrokolloid-Zusammensetzung nach einem der vorhergehenden Ansprüche, bei welchem das Peptid in einer Hydrolyse-Protein-Zusammensetzung enthalten ist.

9. Hydrokolloid-Zusammensetzung nach Anspruch 8, bei welcher die Protein-Zusammensetzung ein oder mehrere Proteine enthält, welche aus Getreideproteinen, Eiweißproteinen, Milchproteinen, Sojaproteinen, Erbsenproteinen, Kartoffelproteinen, Kornproteinen, Gelatinen und Mischungen daraus ausgewählt sind.

10. Hydrokolloid-Zusammensetzung nach einem der vorhergehenden Ansprüche, bei welchem das anionische Polysaccharid aus K-Karagenan, τ-Karagenan, τ/K-Hybrid-Karrageenan, Gellan-Gummi und Mischungen daraus ausgewählt ist.

11. Gelierte oder verdickte, wässrige Zusammensetzung, welche, basierend auf dem Gesamtgewicht von der Zusammensetzung, 0,01 bis 10 Gewichtsprozent von anionischen Polysacchariden und 0,01 bis 40 Gewichtsprozent von Peptiden enthält, welche eine Molekularmasse innerhalb des Bereiches von 0,3 bis 12 kDa haben, wobei die Peptide eine Gesamtmenge von Lysin, Histidin und Arginine zwischen 2,5 bis 40 Mol % enthalten, und zwar basierend auf der Gesamtmenge von Aminosäureresten, welche in den Peptiden enthalten sind, und wobei das Gewichtsverhältnis von anionischen Polysacchariden und den Peptiden in einem Bereich von 1:15 bis 15:1 ist.

12. Nahrungsmittel, welches, basierend auf dem Gesamtgewicht von dem Nahrungsmittel, 0,01 bis 95 Gewichtsprozent von einer gelierten oder verdickten wässrigen Zusammensetzung nach Anspruch 11 enthält.

13. Nahrungsmittel nach Anspruch 12, wobei das Nahrungsmittel nicht mehr als 30 mM von Metallkationen enthält, welche aus Na⁺, K⁺ und Ca²⁺ ausgewählt sind.

14. Kapsel, welche einen aktiven Inhaltsstoff enthält, welcher in einer Matrix enthalten ist, welche 0,5 bis 95 Gewichtsprozent von anionischen Polysacchariden und 0,5 bis 95 Gewichtsprozent von Peptiden enthält, welche eine Molekularmasse innerhalb des Bereiches von 0,3 bis 12 kDa haben, wobei die Peptide eine Gesamtmenge von Lysin, Histidin und Arginine zwischen 2,5 bis 40 Mol % enthalten, und zwar basierend auf der Gesamtmenge von Aminosäureresten, welche in den Peptiden enthalten sind.

15. Verwendung von Peptiden, welche eine Molekularmasse innerhalb des Bereiches von 0,3 bis 12 kDa haben, und welche eine Gesamtmenge von Lysin, Histidin und Arginine zwischen 2,5 bis 40 Mol % enthalten, und zwar basierend auf der Gesamtmenge von Aminosäureresten, welche in den Peptiden enthalten sind, um die Gel- und/oder Viskose-Eigenschaften von anionischen Polysacchariden zu verbessern.

16. Verfahren zum Bereiten einer Hydrokolloid-Zusammensetzung, wobei das Verfahren ein Hydrolysieren einer Protein-Zusammensetzung auf einen derartigen Grad enthält, dass Peptide, welche eine mittlere Molekularmasse innerhalb des Bereiches von 0,3 bis 12 kDa haben, erlangt werden, und Verbinden der Hydrolyse-Protein-Zusammensetzung mit anionischen Polysacchariden in einem Verhältnis im Bereich von 1,0:15,0 bis 15,0:1,0.

17. Hydrokolloid-Zusammensetzung, welche eine Hydrolyse-Protein-Zusammensetzung und anionische Polysaccharide in einem Verhältnis von 1,0:15,0 bis 15,0:1,0 enthält, wobei die Hydrolyse-Protein-Zusammensetzung eine mittlere Molekularmasse im Bereich von 0,3 bis 12 kDa hat, und wobei die Hydrokolloid-Zusammensetzung durch das Verfahren nach Anspruch 16 erlangbar ist.

## Revendications

1. Composition hydrocolloïde comprenant, en poids sec de ladite composition, de 1 à 95 % en poids de polysaccharides anioniques et de 1 à 95 % en poids de peptides ayant un poids moléculaire dans la plage de 0,3 à 12 kDa, dans laquelle lesdits peptides comprennent une quantité totale de lysine, histidine et arginine de 2,5 à 40 % en mol, sur la base de la quantité totale des résidus acides aminés contenus dans les peptides.

2. Composition hydrocolloïde selon la revendication 1, dans laquelle le polysaccharide anionique est choisi parmi les carraghénines, les alginates, l'agar, les pectines, les pectines modifiées, la gomme gellane, la gomme xanthane, le furcellaran, les dérivés cellulosiques, en particulier, la carboxy-méthylcellulose (CMC) et le sulfate de cellulose, le sulfate de dextran, les amidons modifiés, les exopolysaccharides, et leurs mélanges.

3. Composition hydrocolloïde selon la revendication 1 ou 2, dans laquelle le rapport en poids desdits polysaccharides anioniques auxdits peptides est de 1/15 à 15/1.

4. Composition hydrocolloïde selon l'une quelconque des revendications précédentes, dans laquelle le rapport en poids desdits polysaccharides anioniques auxdits peptides est choisi de façon que la composition comprenne une quantité de lysine, histidine et arginine de 0,01 à 75 mmol par gramme de polysaccharides anioniques.

5. Composition hydrocolloïde selon l'une quelconque des revendications précédentes ne comprenant pas plus de 10 mmol de cations métalliques choisis parmi Na⁺, K⁺ et Ca²⁺ par g de polysaccharides anioniques.

6. Composition hydrocolloïde selon l'une quelconque des revendications précédentes, dans laquelle les peptides ont un poids moléculaire dans la plage de 0,5 à 8,0 kDa.

7. Composition hydrocolloïde selon l'une quelconque des revendications précédentes, dans laquelle les peptides comprennent une quantité totale de lysine, histidine et arginine de 5 à 25 % en mol, sur la base de la quantité totale des résidus acides aminés contenus dans lesdits peptides.

8. Composition hydrocolloïde selon l'une quelconque des revendications précédentes, dans laquelle le peptide est contenu dans une composition de protéine(s) hydrolysée.

9. Composition hydrocolloïde selon la revendication 8, dans laquelle la composition de protéine(s) comprend une ou plusieurs protéines choisies parmi les protéines de céréales, les protéines de blanc d'oeuf, les protéines du lait, les protéines de soja, les protéines de pois, les protéines de pomme de terre, les protéines de maïs, les gélatines et leurs mélanges.

10. Composition hydrocolloïde selon l'une quelconque des revendications précédentes, dans laquelle le polysaccharide anionique est choisi parmi la carraghénine K, la carraghénine τ, la carraghénine hybride τ/K_{,} la gomme gellane, et leurs mélanges.

11. Composition aqueuse gélifiée ou épaissie comprenant, sur la base du poids total de la composition, de 0,01 à 10 % en poids de polysaccharides anioniques et de 0,01 à 40 % en poids de peptides ayant un poids moléculaire dans la plage de 0,3 à 12 kDa, dans laquelle lesdits peptides comprennent une quantité totale de lysine, histidine et arginine de 2,5 à 40 % en mol, sur la base de la quantité totale des résidus acides aminés contenus dans les peptides, et dans laquelle le rapport en poids des polysaccharides anioniques aux peptides est de 1/15 à 15/1.

12. Denrée alimentaire comprenant de 0,01 à 95 % en poids, sur la base du poids total de la denrée alimentaire, d'une composition aqueuse gélifiée ou épaissie selon la revendication 11.

13. Denrée alimentaire selon la revendication 12, dans laquelle la denrée alimentaire ne comprend pas plus de 30 mM de cations métalliques choisis parmi Na⁺, K⁺ et Ca²⁺.

14. Encapsulat comprenant un principe actif contenu dans une matrice comprenant de 0,5 à 95 % en poids de polysaccharides anioniques et de 0,5 à 95 % en poids de peptides ayant un poids moléculaire dans la plage de 0,3 à 12 kDa, dans lequel lesdits peptides comprennent une quantité totale de lysine, histidine et arginine de 2,5 à 40 % en mol, sur la base de la quantité totale des résidus acides aminés contenus dans lesdits peptides.

15. Utilisation de peptides ayant un poids moléculaire dans la plage de 0,3 à 12 kDa et comprenant une quantité totale de lysine, histidine et arginine de 2,5 à 40 % en mol, sur la base de la quantité totale des résidus acides aminés contenus dans les peptides, pour améliorer les caractéristiques de gélification et/ou de viscosité des polysaccharides anioniques.

16. Procédé de préparation d'une composition hydrocolloïde, ledit procédé comprenant l'hydrolyse d'une composition de protéine(s) à un degré tel que des peptides ayant un poids moléculaire moyen dans la plage de 0,3 à 12 kDa sont obtenus et la combinaison de ladite composition de protéine(s) hydrolysée avec des polysaccharides anioniques dans un rapport allant de 1,0/15,0 à 15,0/1,0.

17. Composition hydrocolloïde comprenant une composition de protéine(s) hydrolysée et des polysaccharides anioniques dans un rapport allant de 1,0/15,0 à 15,0/1,0, ladite composition de protéine(s) hydrolysée ayant un poids moléculaire moyen dans la plage de 0,3 à 12 kDa et ladite composition hydrocolloïde pouvant être obtenue par le procédé selon la revendication 16.
